(19) Europäisches Patentamt
European Patent Office
Office européen des brevets

(11) EP 3 359 584 B1

(12) **FASCICULE DE BREVET EUROPEEN**

(45) Date de publication et mention de la délivrance du brevet:
**23.06.2021 Bulletin 2021/25**

(21) Numéro de dépôt: **16785245.8**

(22) Date de dépôt: **29.09.2016**

(51) Int Cl.:
***C08G 18/48*** (2006.01) ***C08G 18/75*** (2006.01)
***C08G 18/28*** (2006.01) ***C09D 7/00*** (2018.01)

(86) Numéro de dépôt international:
**PCT/FR2016/052474**

(87) Numéro de publication internationale:
**WO 2017/060586 (13.04.2017 Gazette 2017/15)**

(54) **AGENT EPAISSISSANT POUR SYSTEMES AQUEUX, FORMULATIONS LE CONTENANT ET UTILISATIONS**

VERDICKUNGSMITTEL FÜR WÄSSRIGE SYSTEME, FORMULIERUNGEN DAMIT UND VERWENDUNGEN

THICKENER FOR AQUEOUS SYSTEMS, FORMULATIONS CONTAINING SAME AND USES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **05.10.2015 FR 1559428**

(43) Date de publication de la demande:
**15.08.2018 Bulletin 2018/33**

(73) Titulaire: **Coatex**
**69730 Genay (FR)**

(72) Inventeurs:
- **RUHLMANN, Denis**
**69730 Genay (FR)**
- **SUAU, Jean-Marc**
**69480 Lucenay (FR)**
- **MATTER, Yves**
**69650 Quincieux (FR)**
- **CORFIAS ZUCCALLI, Catherine**
**69100 Villeurbanne (FR)**

(74) Mandataire: **Balmefrezol, Ludovic Francis Pierre**
**Coatex SAS**
**35, rue Ampère**
**69730 Genay (FR)**

(56) Documents cités:
**EP-A1- 2 664 640**
**WO-A1-2012/096125**
**DE-A1-102004 031 786**
**US-A1- 2005 187 342**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 359 584 B1

Printed by Jouve, 75001 PARIS (FR)

## Description

La présente invention concerne de nouveaux épaississants associatifs appartenant à la catégorie des HEUR (Hydrophobically modified Ethyoxylated URethane). Ces produits contiennent un composé associatif comportant un bout de chaîne carboné et une ramification méthyl ou éthyl. La présente invention concerne également des formulations intermédiaires contenant de tels épaississants ainsi que l'utilisation de ces composés comme épaississants dans des compositions finales, par exemple des compositions de peinture.

Les peintures sont constituées de charges et de pigments et d'au moins un polymère organique appelé liant. Outre les charges, les pigments et le liant, une composition de peinture comporte également au moins un solvant (qui est l'eau dans le cas des peintures en phase aqueuse), des additifs pour la rhéologie, des additifs pour la stabilité (stockage, formation du film, UV) et d'autres additifs pour l'obtention de propriétés spécifiques. Le comportement et les propriétés des peintures dépendent de la nature de leurs constituants, notamment du liant, des charges et des pigments, ainsi que des additifs rhéologiques. Elles contiennent généralement un ou plusieurs épaississant(s) dont la fonction est de maîtriser la rhéologie des compositions qui le(s) contiennent, tant au stade de leur fabrication que pendant leur transport, leur stockage ou au cours de leur mise en œuvre. Etant donnée la diversité des contraintes pratiques au niveau de chacune de ces étapes, il est intéressant pour le formulateur de disposer d'une gamme d'épaississants présentant des comportements rhéologiques différents lorsqu'ils sont utilisés dans les compositions finales. En outre, ces épaississants peuvent apporter des propriétés supplémentaires aux compositions, par exemple aux peintures, qui les contiennent.

Parmi tous les épaississants pour peinture, on distingue :

- les épaississants naturels à base de cellulose également appelés éthers cellulosiques, de type HEC ou de type HMHEC (Hydrophobically Modified HEC),
- les épaississants acryliques de type non associatif, appelés ASE (Alkali Swellable Emulsion) et ceux de type associatif, appelés HASE (Hydrophobically modified Alcali Swellable Emulsion) et
- les polyuréthanes épaississants associatifs de type HEUR (Hydrophobically modified Ethyoxylated URethane).

Les polyuréthanes épaississants ou HEUR résultent de la condensation entre un composé de type poly(alkylène glycol), un polyisocyanate et un composé associatif de type alkyle, aryle ou aryalkyle constitué d'un groupe terminal hydrophobe.

Coatex est à l'origine de nombreux travaux de recherche sur les épaississants pour peintures. Par ailleurs, Coatex commercialise les produits de la gamme Coapur®, par exemple les produits Coapur® XS, qui sont des polyuréthanes épaississants non ioniques procurant des profils rhéologiques qui varient entre le type newtonien (viscosité élevée à haut gradient de cisaillement) et/ou le type pseudoplastique (viscosité élevée à bas gradient de cisaillement).

Le document EP 2664640 décrit un polymère alcoxylé hydrosoluble qui est non ionique. Il est préparé en faisant réagir un alcool monovalent hydrophobe, un composé diol linéaire, un oxyde de polyalkylène et un diisocyanate. Ce polymère vise l'épaississement et la transparence pour des compositions cosmétiques.
Le document WO 2012 096125 décrit également un polymère alcoxylé hydrosoluble. Il est préparé par réaction d'un oxyde de polyalkylène, de deux alcools hydrophobes monovalents et d'un diisocyanate. Il vise également l'augmentation la viscosité d'une composition cosmétique.
Le document US 2005 187342 concerne des agents épaississants pour dispersions aqueuses qui sont basés sur une préparation aqueuse de polyuréthanes non ioniques dipersibles ou solubles dans l'eau. Ils sont obtenus à partir d'un isocyanate polyfonctionnel, d'un polyéther-polyol, de 2-(n-butyl)-1-octanol et d'un polyol.
Le document US 2008 108775 décrit une composition aqueuse épaississante comprenant un polyuréthane non-ionique soluble ou dispersible dans l'eau. Ce polyuréthane est obtenu en faisant réagir un polyol hydrophile comprenant des groupes éther ou ester, un composé hydrophobe comprenant au moins une chaîne hydrocarbonée saturée ou insaturée, linéaire ou ramifiée et un diisocyanate.

Les inventeurs ont mis au point un nouveau polyuréthane épaississant qui permet d'augmenter très notablement la viscosité à haut gradient de cisaillement et de conférer, ainsi, à la composition qui le contient un bon comportement dynamique, c'est-à-dire une viscosité élevée à gradient de cisaillement élevé. Cet épaississant peut être classé dans la catégorie des épaississants de type newtonien ou « ICI builder ».
Un épaississant de type « ICI builder » peut être défini comme un produit qui conduit à une augmentation de la viscosité ICI lorsque l'on augmente sa dose au sein de la composition de peinture, sans augmentation importante des viscosités à bas gradient de vitesse (viscosité Brookfield).
Ce nouvel épaississant peut, par exemple, être utilisé seul dans une composition de peinture où il est nécessaire d'avoir une viscosité élevée à haut gradient de cisaillement (par exemple une peinture satinée ou brillante comportant une teneur en liant élevée).
Il peut également être utilisé en combinaison avec un épaississant de type pseudoplastique. Une telle combinaison

permet, ainsi, d'obtenir une composition présentant un bon comportement dynamique lié à la présence de l'épaississant newtonien et un bon comportement statique lié à la présence de l'épaississant de type pseudoplastique.
Un tel épaississant peut être formulé en phase aqueuse et, du fait de sa structure particulière, il permet un épaississement de la composition finale sans nécessiter d'équipement particulier ou d'énergie de cisaillement élevée.
Ce nouvel épaississant présente en bouts de chaînes des groupements hydrophobes qui résultent de la condensation de l'alcool de formule (I) :

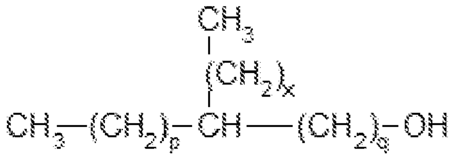

(I)

Le document WO 2011/104599 (Coatex) décrit des polymères modificateurs de rhéologie pour systèmes aqueux, notamment des compositions de peintures. Les épaississants décrits de type épaississant acrylique peuvent contenir, notamment, de l'acrylate d'éthyle (AE), de l'acide méthacrylique (MAA) et des unités monomériques polymérisables de formule :

$$R\text{-}(AO)_m\text{-}(BO)_n\text{-}R'$$

dans laquelle :

- R désigne une fonction insaturée polymérisable, par exemple méthacrylate,
- A et B désignent des groupes alkyles différents l'un de l'autre et ayant de 2 à 4 atomes de carbone, par exemple oxyde d'éthylène et oxyde de propylène,
- m et n sont des entiers inférieurs à 150 dont un au moins est non nul et
- R' est constitué d'au moins un groupement de formule suivante :

  $$CH_3\text{-}(CH_2)_p\text{-}CH(\text{-}CH_3)\text{-}(CH_2)_q\text{-}$$

  dans laquelle :

  - p et q désignent des entiers dont un au moins est non nul et
  - $5 < p + q < 13$.

De tels polymères acryliques présentent un profil rhéologique différent de celui des épaississants de la présente invention. A savoir, ils entrent dans la catégorie des épaississants acryliques de type pseudoplastique (viscosité élevée à bas gradient de cisaillement).

**<u>DEFINITIONS</u>** :

Le terme « HEUR » est un acronyme pour « Hydrophobically modified Ethyoxylated URethane».
Dans la description de la présente invention, à moins qu'il n'en soit indiqué autrement, les pourcentages exprimés représentent des pourcentages en poids et sont exprimés par rapport au poids total de l'élément de référence. Par exemple, lorsqu'il est indiqué qu'un polymère comprend 10 % d'un monomère ou d'un réactif, il est entendu que le polymère comprend 10 % en poids de ce monomère ou réactif par rapport au poids total de ce polymère.
Dans la description de la présente invention, l'expression « au moins un » désigne un ou plusieurs composé(s) (par exemple un ou plusieurs composé(s) alcool(s), un ou plusieurs poly(alkylène(s) glycol(s)), un ou plusieurs polyisocyanate(s)), tel(s) qu'un mélange de 2 à 5 composés.
Par « formulation », on entend l'entité intermédiaire épaississante comprenant l'agent polyuréthane selon l'invention formulé pour être plus facile à utiliser dans la composition finale à épaissir. Par exemple, l'agent épaississant selon l'invention peut être formulé en présence d'eau et d'agents tensioactifs pour être plus facilement coulable/versable (en anglais « pourable ») et plus facile à incorporer dans la composition à épaissir à température ambiante. La viscosité de la formulation avant son incorporation dans la composition aqueuse finale est, par exemple, inférieure à 10 000 mPa.s à 25°C et à 100 tours par minute.

Par « composition », on entend l'entité finale à épaissir ou épaissie comprenant l'agent polyuréthane selon l'invention éventuellement formulé en présence, par exemple, d'eau et d'agents tensioactifs, ainsi que l'ensemble de ses constituants dont la liste dépend de l'application finale. Par exemple, la composition finale comprend des pigments minéraux et des liants, s'il s'agit d'une composition de peinture.

## DESCRIPTION DETAILLEE DE L'INVENTION :

Les polyuréthanes de la présente invention sont des épaississants pour compositions aqueuses, par exemple compositions aqueuses de peinture. Ils permettent d'obtenir des viscosités élevées à haut gradient de cisaillement et de conférer, ainsi, aux compositions un bon comportement dynamique. Ces polyuréthanes épaississants peuvent être classés dans la catégorie des épaississants de type newtonien ou « ICI builder ».

Certaines compositions de peinture, par exemple les peintures satinées ou brillantes qui contiennent peu de pigments (comparativement à une peinture mate par exemple) et beaucoup de liant, doivent présenter une viscosité la plus élevée possible à haut gradient de cisaillement. On parle de viscosité Cone Plan ou de viscosité ICI, notée $\mu_I$ (mPa.s). Le polyuréthane épaississant de la présente invention est tout à fait adapté à ce type de compositions aqueuses. Le polyuréthane épaississant de la présente invention peut également être utilisé en combinaison avec un épaississant de type pseudoplastique. Une telle combinaison permet, ainsi, d'obtenir une composition présentant un bon comportement dynamique lié à la présence de l'épaississant newtonien et un bon comportement statique lié à la présence de l'épaississant de type pseudoplastique. Il est ainsi possible d'ajuster les comportements rhéologiques des formulations aqueuses à bas et haut gradient de cisaillement.

Ce nouvel épaississant présente en bouts de chaînes des groupements hydrophobes spécifiques.

### *Epaississant HEUR :*

Un objet de la présente invention concerne un épaississant appartenant à la catégorie des HEUR (Hydrophobically modified Ethyoxylated URethane). Il s'agit d'un polymère épaississant associatif non ionique pour compositions aqueuses.

Les polyuréthanes épaississants ou HEUR de la présente invention résultent de la réaction entre un réactif qui confère l'associativité et qui est constitué d'un groupe terminal hydrophobe, un composé de type polyol (par exemple poly(alkylène glycol)) et un polyisocyanate. Dans le cadre de la présente invention, on utilise les termes « réaction », « condensation » et « polycondensation » de manière équivalente.

Plus précisément, il s'agit d'un polyuréthane épaississant hydrosoluble résultant exclusivement de la condensation des 3 constituants suivants :

a) d'au moins un alcool de formule (I) :

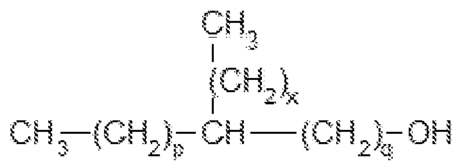

(I)

dans laquelle :

- x représente 0 ou 1,
- p et q sont des entiers dont un au moins est non nul et
- $4 < p + x + q < 7$,

b) d'au moins un poly(alkylène glycol) et
c) d'au moins un polyisocyanate.

Ce sont ces nouveaux polyuréthanes qui permettent, par exemple, d'épaissir une composition de peinture à haut gradient de cisaillement (mesure de la viscosité ICI par exemple).

Le polyuréthane selon la présente invention comporte en tant que constituant a) un composé de formule (I). Un tel composé est appelé, dans le cadre de la présente invention, « composé associatif comportant un bout de chaîne carbonée et une ramification méthyl ou éthyl ».

Par « x représente 0 ou 1 », on entend que la ramification peut respectivement être méthyl ou éthyl.
Etant donné la formule (I), par « 4 < p + x + q < 7 », on entend que la chaîne carbonée comporte entre 7 et 10 atomes de carbone, par exemple 7, 8, 9 ou 10 atomes de carbone.

Ainsi, les composés de formule (I) comportent :

- un bout de chaîne carbonée comportant de 7 à 10 atomes de carbone, par exemple 8 atomes de carbone ou 9 atomes de carbone et

- une ramification en $-CH_3$ ou $-CH_2-CH_3$.

Le polyuréthane épaississant hydrosoluble peut résulter de la condensation d'un ou plusieurs alcool(s) de formule (I).

Selon un mode de réalisation de la présente invention, le polyuréthane est constitué de plusieurs alcools de formule (I). Selon un autre mode de réalisation, ledit polyuréthane est constitué d'un seul alcool de formule (I).

Selon un mode de réalisation de la présente invention, l'alcool a) présente une formule (II) :

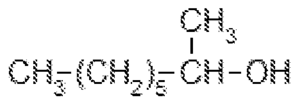

(II)

2-octanol

Selon ce mode de réalisation, les composés de formule (II) comportent :

- un bout de chaîne carbonée comportant 8 atomes de carbone et
- une ramification en $-CH_3$ (ramification méthyl).

Selon un autre mode de réalisation de la présente invention, l'alcool a) présente une formule (III) :

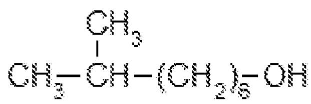

(III)

isononanol

Selon ce mode de réalisation, les composés de formule (III) comportent :

- un bout de chaîne carbonée comportant 9 atomes de carbone et
- une ramification en $-CH_3$ (ramification méthyl).

Par ailleurs, le polyuréthane comporte en tant que constituant b) un poly(alkylène glycol).
Par « poly(alkylène glycol) », on entend un polymère d'un alkylène glycol dérivé d'un oxyde oléfinique. Les chaînes poly(alkylènes glycols) du constituant b) selon la présente invention renferment une proportion de groupes éthylènes-oxys, une proportion de groupes propylènes-oxys et/ou une proportion de groupes butylènes-oxys. Les chaînes poly(alkylènes glycols) selon la présente invention peuvent, par exemple, comprendre une proportion dominante de groupes éthylènes-oxys en association avec une proportion secondaire de groupes propylènes-oxys. Des exemples spécifiques de polymères alkylènes glycols comprennent : les poly(alkylènes glycols) ayant un poids moléculaire moyen de 1 000 g/mol, 4 000 g/mol, 6 000 g/mol et 10 000 g/mol ; les polyéthylènes polypropylènes glycols ayant un pourcentage d'oxyde d'éthylène compris entre 20 % et 80 % en poids et un pourcentage d'oxyde de propylène compris entre 20 % et 80 % en poids.

Selon un aspect de la présente invention, les polyuréthanes résultent de la condensation notamment d'un poly(alkylène glycol) qui est le poly(éthylène glycol). Il peut s'agir, par exemple, d'un poly(éthylène glycol) dont la masse moléculaire varie entre 2 000 g/mol et 20 000 g/mol, par exemple entre 8 000 g/mol et 15 000 g/mol (bornes incluses). A titre d'exemple, on peut citer le poly(éthylène glycol) (ou PEG) de masse moléculaire variant entre 10 000 g/mol et 12 000 g/mol (bornes incluses).

Par ailleurs, le polyuréthane comporte en tant que constituant c) un polyisocyanate. Par « polyisocyanate »,

on entend un composé qui comprend au moins 2 groupes fonctionnels isocyanates -N=C=O.

Selon un aspect de la présente invention, les polyuréthanes résultent de la condensation notamment d'un polyisocyanate qui est choisi dans le groupe consistant en le toluène diisocyanate, les dimères du toluène diisocyanate, les trimères du toluène diisocyanate, le 1,4-butane di-isocyanate, le 1,6-hexane diisocyanate, l'isophorone diisocyanate, le 1,3- cyclohexane diisocyanate, le 1,4- cyclohexane diisocyanate, le 4,4'diisocyanatodicyclohexylméthane, le 1-méthyl-2,4-diisocyanatocyclohexane, le diphényle méthylène diisocyanate (MDI), par exemple le 2,2'-MDI, le 2,4'-MDI, le 4n4'-MDI ou leurs mélanges, le dibenzyl diisocyanate, un mélange du 1-méthyl-2,4-diisocyanatocyclohexane et du 1-méthyl-2,6-diisocyanatocyclohexane, le biuret d'hexaméthylène diisocyanate, les dimères du biuret d'hexaméthylène diisocyanate, les trimères du biuret d'hexaméthylène diisocyanate et un mélange d'au moins deux de ces composés.

Selon l'invention, ledit polyuréthane résulte exclusivement de la condensation des trois constituants suivants :

a) 1 % à 29 % en poids d'au moins un composé de formule (I), (II) et/ou (III),
b) 70 % à 98 % en poids d'au moins un poly(alkylène glycol) et
c) 1 % à 29 % en poids d'au moins un polyisocyanate,

la somme de ces pourcentages massiques étant égale à 100 %.

Selon un aspect de l'invention, ledit polyuréthane résulte exclusivement de la condensation des trois constituants suivants :

a) 3 % à 10 % en poids d'au moins un composé de formule (I), (II) et/ou (III),
b) 80 % à 94 % en poids d'au moins un poly(alkylène glycol) et
c) 3 % à 10 % en poids d'au moins un polyisocyanate,

la somme de ces pourcentages massiques étant égale à 100 %.

La présente invention concerne un polyuréthane épaississant hydrosoluble résultant exclusivement de la condensation des 3 constituants suivants :

a) un alcool de formule (I) :

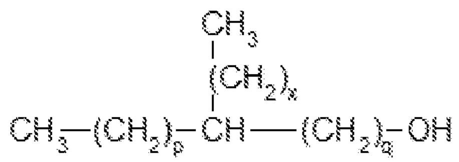

(I)

dans laquelle :

- x représente 0 ou 1,
- p et q sont des entiers dont un au moins est non nul, et
- 4 < p + x + q < 7,

b) un poly(alkylène glycol) et
c) un polyisocyanate.

La fabrication des polyuréthanes, qui appartiennent à la famille des épaississants de type HEUR, est connue de l'homme du métier qui pourra se reporter à l'enseignement des documents cités auparavant dans l'arrière-plan technologique de la présente invention. Un autre objet de la présente invention concerne également un procédé de préparation d'un polyuréthane tel que décrit ci-dessus, ledit procédé consistant en une condensation de ses différents constituants.

***Formulation de l'épaississant HEUR :***

Le polyuréthane selon l'invention peut être formulé ou co-formulé avec d'autres constituants ou composants. Ainsi, la présente invention concerne également une formulation aqueuse comprenant un polyuréthane selon l'invention, tel que décrit ci-dessus.
Cette formulation aqueuse épaississante est destinée à être incorporée dans une composition finale, par exemple une peinture, une sauce de couchage ou une composition détergente.

Le polyuréthane selon l'invention peut être co-formulé en présence d'eau.
Selon un mode de réalisation, ladite formulation aqueuse selon l'invention consiste en :

1) 5 % à 50 % en poids d'au moins un polyuréthane selon l'invention, tel que décrit ci-dessus et

2) 50 % à 95 % en poids d'eau,

la somme de ces pourcentages massiques étant égale à 100 %.
Selon un autre mode de réalisation, ladite formulation aqueuse selon l'invention consiste en :

1) 5 % à 25 % en poids d'au moins un polyuréthane selon l'invention, tel que décrit ci-dessus et

2) 75 % à 95 % en poids d'eau,

la somme de ces pourcentages massiques étant égale à 100 %.

Le polyuréthane selon l'invention peut être co-formulé dans l'eau, en présence d'au moins un agent tensioactif. Cet agent tensioactif permet de formuler l'épaississant sous la forme d'une solution aqueuse liquide moins visqueuse qui peut ainsi être plus facilement mise en œuvre par le formulateur.

Ainsi, selon un mode de réalisation de la présente invention, ladite formulation aqueuse comprend un polyuréthane, tel que décrit ci-dessus, ainsi que de l'eau et au moins un agent tensioactif.
Par « tensioactif » ou « agent tensioactif », on entend une molécule ou un polymère constitué d'au moins une partie hydrophile et d'au moins une partie hydrophobe. L'agent tensioactif utilisé dans le cadre de la présente invention peut être de nature différente, par exemple il peut être anionique ou non ionique.
Cet agent tensioactif peut être sélectionné parmi les classes d'agents tensioactifs ioniques (dans ce cas de préférence anioniques) et/ou non ioniques et/ou mixtes (comportant dans la même molécule une structure non ionique et anionique). L'agent tensioactif préféré est composé d'au moins un agent tensioactif sélectionné dans la classe d'agents tensioactifs non ioniques, éventuellement en présence d'un agent tensioactif anionique.
Parmi les agents tensioactifs anioniques convenables, on peut citer les sels de sodium, lithium, potassium, ammonium ou de magnésium dérivés des alkyles éthers sulfates avec alkyle(s) variant de C6 à C12, en configuration linéaire, iso, oxo, géminé, cyclique ou aromatique, ou des alkyles sulfates en C12, des esters alkyles phosphates ou les dialkyles sulfosuccinates. Les agents tensioactifs anioniques sont, de préférence, utilisés avec au moins un agent tensioactif non ionique.

Comme exemples d'agents tensioactifs mixtes, on peut citer les sulfonates d'alkyles phénols alkoxylés. Les agents tensioactifs non ioniques peuvent être utilisés seuls ou en combinaison avec un agent tensioactif anionique. Comme exemples préférés d'agents tensioactifs non ioniques convenables, on peut citer : les alcools en C4-C18 éthoxylés (2 à 15 OE), les alcools de Guerbet en C4-C18 éthoxylés (2 à 40 OE), les alcools monobranchés en C10-C18 éthoxylés (2 à 40 OE), les esters de sorbitol en C18, les esters de sorbitol éthoxylés (2 à 20 motifs OE), les acides en C4-C18 éthoxylés (inférieurs à 15 OE), l'huile de ricin éthoxylée (30 à 40 OE), l'huile de ricin hydrogénée éthoxylée (7 à 60 OE), les esters tels que le palmitate de glycérol, le stéarate de glycérol, le stéarate d'éthylène glycol, le stéarate de diéthylène glycol, le stéarate de propylène glycol, le stéarate de polyéthylène glycol 200 et les esters en C18 éthoxylés (2 à 15 OE). Les chaînes hydrophobes peuvent correspondre à des structures linéaires, iso, oxo, cycliques ou aromatiques.

Selon un mode de réalisation, la formulation comprend au moins un agent tensioactif non ionique éventuellement combiné avec au moins un agent tensioactif anionique, à une teneur totale en poids allant de 0,1 % à 40 % en poids, par exemple de 5 % à 20 % en poids ou de 10 % à 17 % en poids. Dans ce cas, le rapport en poids entre les deux agents tensioactifs peut, par exemple, varier entre 25/75 et 75/25.

Selon un mode de réalisation de la présente invention, le polyuréthane de la présente invention est formulé en présence de plus de deux agents tensioactifs, par exemple trois ou quatre.

Selon un mode de réalisation, ladite formulation aqueuse selon l'invention consiste en :

1) 2 % à 50 % en poids d'au moins un polyuréthane selon l'invention, tel que décrit ci-dessus, de préférence 5 % à 30 % en poids,
2) 0,1 % à 40 % en poids d'au moins un agent tensioactif, de préférence 2 % à 30 % en poids et
3) 10 % à 93 % en poids d'eau, de préférence 40 % à 85 % en poids,

la somme de ces pourcentages massiques étant égale à 100 %.

Le polyuréthane selon l'invention peut être formulé dans un solvant miscible à l'eau. La raison principale de l'ajout d'un co-solvant organique est d'abaisser la viscosité de ce polyuréthane dans l'eau, afin de faciliter la manipulation.

Le polyuréthane est, par exemple, formulé avec un ou plusieurs solvant(s) polaire(s) appartenant notamment au groupe constitué par l'eau, le méthanol, l'éthanol, le propanol, l'isopropanol, les butanols, l'acétone, le tétrahydrofurane ou leurs mélanges.

Deux exemples particuliers de solvants organiques miscibles à l'eau sont :

- l'éther de diéthylène glycol monobutylique (également connu sous le nom de Butyl Carbitol™) ou d'éthylène ou de propylène glycol et
- l'éther de butylène glycol.

La viscosité du polyuréthane en l'état, avant son incorporation dans une composition de peinture par exemple, est avantageusement inférieure à 10 000 mPa.s à 25°C et à 100 tours par minute, de sorte qu'il est plus facile à verser à partir du récipient de stockage et plus rapidement incorporé dans la composition à épaissir à température ambiante. Le solvant miscible à l'eau choisi pour de telles compositions commerciales a, jusqu'à ce jour, exclusivement été un solvant organique.

Le polyuréthane selon l'invention peut être co-formulé dans l'eau, en présence d'un agent de coalescence. De manière équivalente à un solvant, l'agent de coalescence permet de formuler l'épaississant sous forme d'une solution aqueuse liquide moins visqueuse qui peut ainsi être plus facilement mise en œuvre par le formulateur.

Selon un mode de réalisation, ladite formulation aqueuse selon l'invention consiste en :

1) 5 % à 50 % en poids d'au moins un polyuréthane selon l'invention, tel que décrit ci-dessus,
2) 5 % à 30 % en poids d'au moins un solvant et/ou agent de coalescence et
3) 20 % à 75 % en poids d'eau,

la somme de ces pourcentages massiques étant égale à 100 %.

Selon un aspect de l'invention, la formulation aqueuse comprend, en outre, au moins un additif sélectionné dans le groupe consistant en un biocide, un solvant, un agent anti-mousse, un régulateur de pH, un agent de coalescence, un agent encapsulant et leurs mélanges.

Par « biocide », on entend une substance chimique destinée à détruire, repousser ou rendre inoffensifs les organismes nuisibles, à en prévenir l'action ou à les combattre de toutes autres manières, par une action chimique ou biologique.

Par « agent anti-mousse », on entend une substance ou une formulation destinée à détruire les bulles d'air au sein d'un milieu liquide homogène ou hétérogène (ou à sa surface) ou à prévenir leur formation.

Par « régulateur de pH » ou « agent régulateur de pH », on entend un composé chimique qui permet d'ajuster le pH à la valeur attendue. Par exemple, l'agent régulateur de pH peut augmenter le pH, c'est le cas des bases telles que NaOH. Alternativement, l'agent régulateur de pH peut diminuer le pH, c'est le cas des acides.

Par « agent de coalescence », on entend un agent utilisé dans les peintures qui permet d'abaisser la Température Minimum de Formation du Film (TMFF ou MFFT pour Minimum Film Formation Temperature) de peinture à une température adaptée aux conditions d'application souhaitées (par exemple une TMFF de 5°C pour une application à l'extérieur). A titre d'exemples d'agents coalescents selon l'invention, on peut citer le propylène glycol, le butyl glycol, le 2,2,4-triméthyl-1,3-pentanediol monoisobutyrate ou le 2,2,4-triméthyl-1,3-pentanediol diisobutyrate, le 2,2,4-triméthyl-1,3-pentanediol monoisobutyrate, les dérivés d'éthers de glycol de type Dowanol®.

Par « agent encapsulant », on entend un agent créant un environnement hydrophobe, par exemple une cage de solvatation. On cite en particulier, comme agent encapsulant, la cyclodextrine.

Selon un mode de réalisation, ladite formulation aqueuse selon l'invention consiste en :

1) 2 % à 50 % en poids d'au moins un polyuréthane selon l'invention, tel que décrit ci-dessus, de préférence 2 % à 30 % en poids,
2) 0,1 % à 40 % en poids d'au moins un agent tensioactif, de préférence 5 % à 30 % en poids,
3) 10 % à 93 % en poids d'eau, de préférence 40 % à 85 % en poids et
4) 0 % à 5 % en poids d'au moins un autre additif choisi dans le groupe consistant en un biocide, un solvant, un agent anti-mousse, un régulateur de pH, un agent de coalescence, un agent encapsulant et leurs mélanges, de préférence 0,5 % à 4 % en poids,

la somme de ces pourcentages massiques étant égale à 100 %.

***<u>Composition aqueuse finale et utilisations du polyuréthane</u>*** *:*

Un objet de la présente invention consiste en une composition aqueuse comprenant un polyuréthane selon

l'invention ou une formulation aqueuse épaississante selon l'invention, ladite composition aqueuse finale étant sélectionnée dans le groupe consistant en une peinture, un enduit, un revêtement épais, un revêtement d'imperméabilisation, une laque, un vernis, une encre, une suspension minérale (slurry), une sauce de couchage papetière, une composition cosmétique et une composition détergente.

Ladite composition est épaissie au moyen d'un polyuréthane ou d'une formulation aqueuse épaississante selon l'invention.

Par ailleurs, la présente invention concerne également l'utilisation d'un polyuréthane selon l'invention ou d'une formulation aqueuse selon l'invention, pour épaissir une composition aqueuse, ladite composition étant sélectionnée dans le groupe consistant en une laque, un vernis, une peinture, un enduit, un revêtement épais, un revêtement d'imperméabilisation, une encre, une suspension minérale (slurry), une sauce de couchage papetière, une composition cosmétique et une composition détergente.

Plus spécifiquement, selon un mode de réalisation, la présente invention concerne l'utilisation d'un polyuréthane selon l'invention ou d'une formulation aqueuse selon l'invention, pour augmenter la viscosité de la composition aqueuse à haut gradient de cisaillement.

Par « augmenter la viscosité de la composition aqueuse à haut gradient de cisaillement », on entend que la viscosité à haut gradient de cisaillement de la composition aqueuse à une dose donnée de polyuréthane est supérieure à celle de la même composition aqueuse à une dose inférieure du même polyuréthane. En d'autres mots, augmenter la dose de polyuréthane dans la composition aqueuse conduit à augmenter la viscosité à haut gradient de cisaillement de cette composition.

Selon un mode de réalisation, l'utilisation d'un polyuréthane selon l'invention ou d'une formulation aqueuse selon l'invention, permet d'augmenter sélectivement la viscosité de la composition aqueuse à haut gradient de cisaillement (par exemple viscosité ICI), c'est à dire sans augmentation importante des viscosités à bas et moyen gradient de vitesse (par exemple viscosités Brookfield et Stormer). En d'autres mots, augmenter la dose de polyuréthane dans la composition aqueuse conduit à augmenter la viscosité à haut gradient de cisaillement de cette composition, sans que cette augmentation de dose ne conduise à augmenter de manière importante les viscosités à bas et moyen gradient de cisaillement.

Les inventeurs ont mis au point un nouveau polyuréthane épaississant qui permet d'augmenter très notablement la viscosité à haut gradient de cisaillement et de conférer, ainsi, à la composition qui le contient un bon comportement dynamique, c'est-à-dire une viscosité élevée à gradient de cisaillement élevé. Cet épaississant peut être classé dans la catégorie des épaississants de type newtonien ou « ICI builder ».

Un épaississant de type « ICI builder » peut être défini comme un produit qui conduit à une augmentation de la viscosité ICI lorsque l'on augmente sa dose au sein de la composition de peinture, sans augmentation importante des viscosités à bas et moyen gradient de vitesse (viscosités Brookfield et Stormer).

Ce nouvel épaississant peut, par exemple, être utilisé seul dans une composition de peinture où il est nécessaire d'avoir une viscosité élevée à haut gradient de cisaillement (par exemple une peinture satinée ou brillante comportant une teneur en liant élevée).

Selon un mode de réalisation, la composition aqueuse à épaissir est de type peinture brillante, peinture semi-brillante, peinture satinée ou toutes autres peintures à basse Concentration Pigmentaire Volumique (CPV).

La « concentration pigmentaire volumique » est définie par la formule suivante :

$$CPV\ (\%) = 100 \times V_c / (V_c+V_l)$$

avec $V_c$ qui représente le volume des charges minérales et

$V_l$ qui représente le volume de liants dans la formulation de peinture.

Selon un autre mode de réalisation, la composition aqueuse à épaissir est de type peinture à moyenne ou haute Concentration Pigmentaire Volumique (CPV), variant entre la peinture coquille d'œuf (« egg-shell » en anglais) et la peinture mate. Dans ce cas, le polyuréthane épaississant de la présente invention peut être associé à un autre épaississant présentant un profil pseudoplastique.

Selon un mode de réalisation de la présente invention, ledit polyuréthane ou ladite formulation aqueuse de polyuréthane est utilisé comme agent d'étalement-tendu de ladite composition aqueuse. Le polyuréthane selon l'invention permet, par exemple, d'augmenter la valeur d'étalement-tendu de la peinture qui le contient, c'est-à-dire la capacité auto-lissante de la peinture lors de l'application. Cette valeur peut, par exemple, être mesurée sur une carte de contraste Leneta, norme ASTM D4062, « flow & levelling » en anglais.

Selon un mode de réalisation de la présente invention, l'utilisation dudit polyuréthane ou de ladite formulation aqueuse de polyuréthane dans une composition aqueuse de peinture permet notamment d'améliorer les propriétés applicatives suivantes :

- la résistance à l'application (en anglais « brush drag »),
- l'effet garnissant (en anglais « film build »),
- le pouvoir masquant grâce à une dépose plus régulière (en anglais « hiding power »), et
- la diminution des éclaboussures (en anglais « spatter resistance »).

Selon un aspect de la présente invention, la composition aqueuse comprend de 0,02 % à 5 % en poids de matière active dudit épaississant.

Selon un autre aspect de la présente invention, la formulation aqueuse comprend de 0,05 % à 2 % en poids de matière active dudit épaississant.

Par « poids de matière active », on entend le poids sec de polyuréthane selon l'invention, indépendamment des ingrédients de co-formulation.

Selon un autre aspect encore de la présente invention, la composition aqueuse comprend au moins une charge minérale sélectionnée dans le groupe consistant en le carbonate de calcium, le kaolin, le talc et le silicate et/ou au moins un pigment sélectionné dans le groupe consistant en le dioxyde de titane, l'oxyde de fer et le zinc.

Selon un aspect de l'invention, la composition aqueuse est une peinture et comprend au moins un agent dispersant, au moins une charge ou un pigment minéral, au moins un liant, au moins un biocide, au moins un agent anti-mousse et éventuellement un agent tensioactif, un agent de surface et/ou un agent de coalescence, un solvant.
Les exemples qui suivent permettent de mieux appréhender la présente invention, sans en limiter la portée.

## EXEMPLES

On détermine la viscosité des formulations de test ou des compositions de peinture à différents gradients de vitesse :

- à faible gradient de vitesse : la viscosité Brookfield (Bk) qui est mesurée à l'aide d'un viscosimètre Brookfield de type RVT, dans le flacon non agité, à une température de 25°C et à deux vitesses de rotation de 10 et 100 tours par minute avec le mobile adéquat.

La lecture est effectuée après 1 minute de rotation. On obtient, ainsi, 2 mesures de viscosité Brookfield respectivement notées $\mu_{Bk10}$ et $\mu_{Bk100}$ (mPa.s),

- à moyen gradient de vitesse : la viscosité Stormer, notée $\mu_S$ (Krebs Units ou KU) et
- à haut gradient de vitesse : la viscosité Cone Plan ou viscosité ICI, notée $\mu_I$ (Poise ou P).

### Exemple 1 :

Cet exemple illustre l'utilisation d'un épaississant selon l'invention dans une formulation de peinture satinée, aqueuse, sans solvant, dont la constitution est donnée dans le tableau 1 ci-dessous.
Il illustre le pouvoir épaississant d'un polyuréthane selon l'invention (essais 1-3 et 2-3), mettant en œuvre un composé de formule (III). Parallèlement, cet exemple illustre aussi des polyuréthanes hors invention (essais 1-1 et 2-1) et un épaississant acrylique HASE selon (essais 1-2 et 2-2).

<u>Essais 1-1 et 2-1</u> (hors invention) :

Ledit polyuréthane résulte de la condensation de, exprimé en pourcentage en poids par rapport au poids total du polyuréthane :

- 2,7 % en poids d'un alcool linéaire non éthoxylé de formule $CH_3-(CH_2)_7-OH$,
- 92,8 % en poids de PEG 10 000 et
- 4,5 % en poids d'isophorone diisocyanate (IPDI).

<u>Essais 1-2 et 2-2</u> (selon l'invention) :

Ledit polyuréthane résulte de la condensation de, exprimé en pourcentage en poids par rapport au poids total du polyuréthane :

- 3,5 % en poids d'un composé de formule (II) :

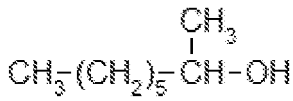

(II)

- 90,5 % en poids de PEG 10 000 et
- 6,0 % en poids d'isophorone diisocyanate (IPDI).

Essais 1-3 et 2-3 (selon l'invention) :

Ledit polyuréthane résulte de la condensation de, exprimé en pourcentage en poids par rapport au poids total du polyuréthane :

- 3,4 % en poids d'un composé de formule (III) :

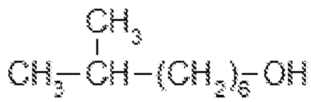

(III)

- 91, 3 % en poids de PEG 10 000 et
- 5,3 % en poids d'isophorone diisocyanate (IPDI).

Les polyuréthanes sont formulés dans l'eau en présence d'un agent tensioactif qui est une coupe C8-C10 d'un alcool gras alkoxylé (Simulsol®OX1008). Les ratios PU/tensioactif/eau sont 20/5/75.

Tous les résultats ont été regroupés dans les Tableaux 2 et 3 ci-dessous.

Pour chacun des essais, on a déterminé les viscosités $\mu_{Bk10}$, $\mu_{Bk100}$, $\mu_I$ (en P) et $\mu_S$ (en Krebs Units mesurées avec le module standard), selon les méthodes décrites ci-dessus à T = 0 et à T = 24 h, à température ambiante.

**Tableau 1**

| Constituant de la peinture Masse (g) | |
|---|---|
| Eau | 99,45 |
| Dispersant (Coadis® BR3, Coatex) | 3,9 |
| Biocide (Acticide® MBS, Thor) | 1,3 |
| Agent anti-mousse (Airex® 901W, Tego) | 1,31 |
| $NH_4OH$ (28 %) | 0,5 |
| $TiO_2$ (RHD2® Hunstman) | 122,2 |
| $CaCO_3$ (Omyacoat® 850OG, Omva) | 84,5 |
| Liant (Acronal 290D, BASF) | 270,6 |
| Mono propylène glycol | 6,5 |
| Texanol® (Eastman) | 6,5 |
| Agent anti-mousse (Tego® 825, Tego) | 0,65 |
| *Epaississant PU* (*selon les essais*) | Série 1 : 28,6<br>Série 2 : variable (voir Tableau 3) |
| Eau | q.s.p. 650 g au total |

**Tableau 2**

| | | Essai 1-1 | Essai 1-2 | Essai 1-3 |
|---|---|---|---|---|
| | | HINV | INV | INV |
| Dose (% en poids / formule totale) | | 0,88 | | |
| T = 0 | $\mu_{Bk10}$ | 2 660 | 1 730 | 1 795 |
| | $\mu_{Bk100}$ | 1 584 | 957 | 1 110 |
| | $\mu_S$ | 95 | 81 | 85 |
| | $\mu_I$ | 2,5 | 2,2 | 2,0 |
| T = 24 h | $\mu_{Bk10}$ | 3 320 | 1 730 | 2 110 |
| | $\mu_{Bk100}$ | 2018 | 1 012 | 1 333 |
| | $\mu_S$ | 102 | 84 | 91 |
| | $\mu_I$ | 2,5 | 2,2 | 2,0 |

**Tableau 3**

| | | Essai 2-1 | Essai 2-2 | Essai 2-3 |
|---|---|---|---|---|
| | | HINV | INV | INV |
| Dose (% en poids / formule totale) | | 1,44 | 1,44 | 1,44 |
| T = 0 | $\mu_{Bk10}$ | 4 560 | 2 560 | 2 560 |
| | $\mu_{Bk100}$ | 2 580 | 1 360 | 1 492 |
| | $\mu_S$ | 118 | 90 | 97 |
| | $\mu_I$ | 3,9 | 3,9 | 3 |
| T = 24h | $\mu_{Bk10}$ | 5 800 | 3 240 | 3 240 |
| | $\mu_{Bk100}$ | 3 492 | 1 872 | 2 108 |
| | $\mu_S$ | 126 | 99 | 109 |
| | $\mu_I$ | 4 | 4 | 3,2 |
| HINV : Hors INVention INV : Selon l'INVention | | | | |

Les polyuréthanes selon la présente invention (essais 1-2, 2-2, essais 1-3 et 2-3) présentent un profil rhéologique de type newtonien : viscosité faible à bas et moyen gradient de cisaillement.

Par comparaison des résultats présentés des essais 1-3 et 2-3 (selon l'invention), on constate un épaississement significativement amélioré à haut gradient de vitesse ($\mu_I$) dans la formulation de peinture alors que les viscosités Brookfield et Stormer évoluent plus modérément, cela est caractéristique d'un épaississant newtonien, de type « ICI builder », qui permet une augmentation sélective de la viscosité ICI en fonction de la dose.

Le polyuréthane de la présente invention offre donc un bon compromis entre comportement newtonien (qui permet d'obtenir des viscosités faibles à bas gradient et moyen gradient de cisaillement) et caractéristique « ICI builder » qui permet une augmentation sélective de la viscosité ICI en fonction de la dose utilisée. Le formulateur peut, ainsi, ajuster la dose d'épaississant en fonction du comportement rhéologique souhaité à haut gradient de cisaillement.

Le polyuréthane hors invention de l'essai 1-1 permet d'obtenir un épaississement à haut gradient de vitesse ($\mu_I$) de même ordre de grandeur que celui obtenu avec les polyuréthanes selon l'invention des essais 1-2 et 1-3. Néanmoins, on constate que les viscosités Brookfield et Stormer obtenues avec le polyuréthane hors invention de l'essai 1-1 sont globalement plus élevées que celles obtenues avec le polyuréthane selon l'invention des essais 1-2 et 1-3 à une dose identique (0,88 % en poids par rapport au poids total de la composition).

Le polyuréthane des essais 1-1 et 2-1 génère une viscosité ICI modulable en fonction de la dose ajoutée dans la formule, mais elle est couplée à des viscosités trop élevées à bas et à moyens gradients de vitesse. Le profil de ce

polyuréthane n'est pas suffisamment newtonien.

## Revendications

1. Polyuréthane épaississant hydrosoluble résultant exclusivement de la condensation :

   a) d'au moins un alcool de formule (I) :

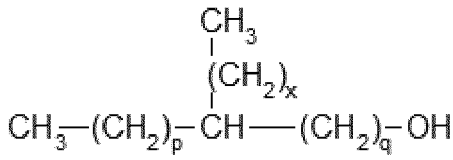

(I)

   dans laquelle :

   - x représente 0 ou 1,
   - p et q sont des entiers dont un au moins est non nul et
   - 4 < p + x + q < 7,

   b) d'au moins un poly(alkylène glycol) et
   c) d'au moins un polyisocyanate.

2. Polyuréthane selon la revendication 1, dans lequel l'alcool a) présente une formule (II) :

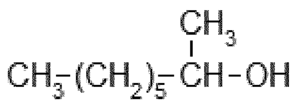

(II)

3. Polyuréthane selon la revendication 1, dans lequel l'alcool a) présente une formule (III) :

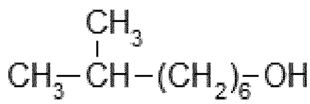

(III)

4. Polyuréthane selon l'une quelconque des revendications précédentes, résultant de la condensation de :

   a) 1 % à 29 % en poids d'au moins un composé de formule (I), (II) et/ou (III),
   b) 70 % à 98 % en poids d'au moins un poly(alkylène glycol) et
   c) 1 % à 29 % en poids d'au moins un polyisocyanate,

   la somme de ces pourcentages massiques étant égale à 100 %.

5. Polyuréthane selon l'une quelconque des revendications précédentes, selon lequel le poly(alkylène glycol) est un poly(éthylène glycol) dont la masse moléculaire varie entre 2 000 g/mol et 20 000 g/mol.

6. Formulation aqueuse comprenant un polyuréthane selon l'une quelconque des revendications 1 à 5.

7. Formulation aqueuse selon la revendication 6, comprenant, en outre, de l'eau et au moins un agent tensioactif.

**8.** Formulation aqueuse selon la revendication 6 ou 7, comprenant, en outre, au moins un additif sélectionné dans le groupe consistant en un biocide, un solvant, un agent anti-mousse, un régulateur de pH, un agent de coalescence, un agent encapsulant et leurs mélanges.

**9.** Formulation aqueuse selon l'une quelconque des revendications 6 à 8, consistant en :

1) 2 % à 50 % en poids d'au moins un polyuréthane selon l'une quelconque des revendications 1 à 5,
2) 0,1 % à 40 % en poids d'au moins un agent tensioactif,
3) 10 % à 93 % en poids d'eau et
4) 0 % à 5 % en poids d'au moins un autre additif choisi dans le groupe consistant en un biocide, un solvant, un agent anti-mousse, un régulateur de pH, un agent de coalescence, un agent encapsulant et leurs mélanges,

la somme de ces pourcentages massiques étant égale à 100 %.

**10.** Utilisation d'un polyuréthane selon l'une quelconque des revendications 1 à 5 ou d'une formulation aqueuse selon l'une quelconque des revendications 6 à 9, pour épaissir une composition aqueuse, ladite composition étant sélectionnée dans le groupe consistant en une laque, un vernis, une peinture, un enduit, un revêtement épais, un revêtement d'imperméabilisation, une encre, une suspension minérale (slurry), une sauce de couchage papetière, une composition cosmétique et une composition détergente.

**11.** Utilisation selon la revendication 10, d'un polyuréthane ou d'une formulation aqueuse de polyuréthane comme agent d'étalement-tendu de ladite composition aqueuse.

## Patentansprüche

**1.** Wasserlösliches verdickendes Polyurethan, ausschließlich hergestellt durch die Kondensation von:

a) mindestens einem Alkohol mit folgender Formel (I):

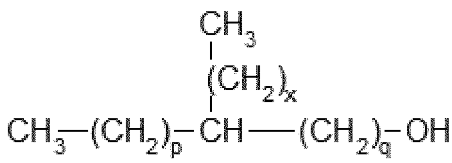

(I)

wobei:

- x gleich 0 oder 1 ist,
- p und q Ganzzahlen sind, von denen mindestens eine Zahl ungleich Null ist, und
- $4 < p + x + q < 7$,

b) mindestens einem Poly(alkylenglykol) und
c) mindestens einem Polyisocyanat.

**2.** Polyurethan nach Anspruch 1, bei dem der Alkohol a) folgende Formel (II) aufweist:

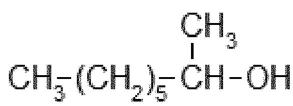

(II)

**3.** Polyurethan nach Anspruch 1, bei dem der Alkohol a) folgende Formel (III) aufweist:

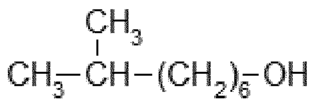

(III)

**4.** Polyurethan nach einem der vorstehenden Ansprüche, hergestellt durch die Kondensation von:

a) 1 bis 29 Gew.-% mindestens einer Verbindung mit der Formel (I), (II) und/oder (III),
b) 70 bis 98 Gew.-% mindestens eines Poly(alkylenglykols) und
c) 1 bis 29 Gew.-% mindestens eines Polyisocyanats,

wobei die Summe dieser Massenanteile gleich 100 % ist.

**5.** Polyurethan nach einem der vorstehenden Ansprüche, bei dem das Poly(alkylenglykol) ein Poly(ethylenglykol) ist, dessen molekulare Masse zwischen 2.000 g/mol und 20.000 g/mol variiert.

**6.** Wässrige Formulierung mit einem Polyurethan nach einem der vorstehenden Ansprüche 1 bis 5.

**7.** Wässrige Formulierung nach Anspruch 6, die zusätzlich Wasser und mindestens ein Tensid enthält.

**8.** Wässrige Formulierung nach Anspruch 6 oder 7, die zusätzlich mindestens ein Additiv enthält, das aus der Gruppe bestehend aus einem Biozid, einem Lösungsmittel, einem Antischaummittel, einem pH-Regulator, einem Koaleszenzmittel, einem Verkapselungsmittel und deren Mischungen ausgewählt ist.

**9.** Wässrige Formulierung nach einem der vorstehenden Ansprüche 6 bis 8, bestehend aus:

1) 2 bis 50 Gew.-% mindestens eines Polyurethans nach einem der vorstehenden Ansprüche 1 bis 5,
2) 0,1 bis 40 Gew.-% mindestens eines Tensids,
3) 10 bis 93 Gew.-% Wasser und
4) 0 bis 5 Gew.-% mindestens eines anderen Additivs, das aus der Gruppe bestehend aus einem Biozid, einem Lösungsmittel, einem Antischaummittel, einem pH-Regulator, einem Koaleszenzmittel, einem Verkapselungsmittel und deren Mischungen ausgewählt ist,

wobei die Summe dieser Massenanteile gleich 100 % ist.

**10.** Verwendung eines Polyurethans nach einem der vorstehenden Ansprüche 1 bis 5 oder einer wässrigen Formulierung nach einem der vorstehenden Ansprüche 6 bis 9 zum Verdicken einer wässrigen Zusammensetzung, wobei diese Zusammensetzung aus der Gruppe bestehend aus einem Lack, einer Glasur, einer Farbe, einem Überzug, einer dicken Beschichtung, einer Abdichtungsbeschichtung, einer Druckfarbe, einer mineralischen Suspension (Slurry), einer Streichmasse für die Papierindustrie, einer kosmetischen Zusammensetzung und einer Detergenszusammensetzung ausgewählt ist.

**11.** Verwendung nach Anspruch 10 eines Polyurethans oder einer wässrigen Polyurethan-Formulierung als Verlaufsmittel dieser wässrigen Zusammensetzung.

## Claims

**1.** A water-soluble thickening polyurethane resulting from the exclusive condensation:

a) of at least one alcohol of formula (I):

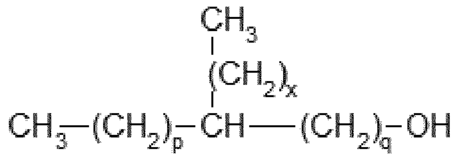

(I)

in which:

- x represents 0 or 1,
- p and q are integers, at least one of which is non-zero and
- $4 < p + x + q < 7$,

b) of at least one poly(alkylene glycol) and
c) of at least one polyisocyanate.

**2.** The polyurethane according to claim 1, wherein the alcohol a) has a formula (II):

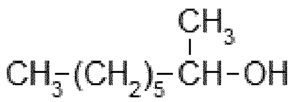

(II)

**3.** The polyurethane according to claim 1, wherein the alcohol a) has a formula (III):

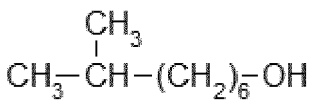

(III)

**4.** The polyurethane according to any one of the preceding claims, resulting from the condensation of:

a) 1% to 29% by weight of at least one compound of formula (I), (II) and/or (III),
b) 70% to 98% by weight of at least one poly(alkylene glycol) and
c) 1% to 29% by weight of at least one polyisocyanate,

the sum of these mass percentages being equal to 100%.

**5.** The polyurethane according to any one of the preceding claims, wherein the poly(alkylene glycol) is a poly(ethylene glycol) of which the molecular mass ranges between 2000 g/mol and 20 000 g/mol.

**6.** An aqueous formulation comprising a polyurethane according to any one of claims 1 to 5.

**7.** The aqueous formulation according to claim 6, also comprising water and at least one surface-active agent.

**8.** The aqueous formulation according to claim 6 or 7, also comprising at least one additive selected from the group consisting of a biocide, a solvent, an anti-foaming agent, a pH regulator, a coalescent agent, an encapsulating agent and mixtures thereof.

**9.** The aqueous formulation according to any one of claims 6 to 8, consisting of:

1) 2% to 50% by weight of at least one polyurethane as claimed in any one of claims 1 to 5,
2) 0.1% to 40% by weight of at least one surface-active agent,
3) 10% to 93% by weight of water, and
4) 0% to 5% by weight of at least one other additive chosen from the group consisting of a biocide, a solvent,

an anti-foaming agent, a pH regulator, a coalescent agent, an encapsulating agent and mixtures thereof,

the sum of these mass percentages being equal to 100%.

**10.** The use of a polyurethane according to any one of claims 1 to 5 or of an aqueous formulation as claimed in any one of claims 6 to 9, for thickening an aqueous composition, said composition being selected from the group consisting of a lacquer, a varnish, a paint, a putty, a thick coating, a waterproofing coating, an ink, a slurry, a paper coating color, a cosmetic composition and a detergent composition.

**11.** The use according to claim 10, of a polyurethane or of an aqueous polyurethane formulation, as a leveling agent for said aqueous composition.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 2664640 A
- WO 2012096125 A
- US 2005187342 A
- US 2008108775 A
- WO 2011104599 A